# EUROPEAN PATENT APPLICATION

(11) **EP 3 925 967 A1**
(43) Date of publication of application: **22.12.2021**
(21) Application number: 21167594.7
(22) Date of filing: 09.04.2021
(51) Int. Cl.: C07K 1/04, C08G 85/00

(54) **OXIME-CONTAINING POLYMER**

(30) Priority: 17.06.2020 WO PCT/RU2020/000299
(71) Applicant: JSC Cytomed, 197375 St. Petersburg (RU)
(72) Inventor: BUROV, Sergej Vladimirovich, St. Petersburg (RU); LEKO, Maria Viktorovna, St. Petersburg (RU)
(74) Representative: Jeck, Anton

(57) **Abstract**

The invention relates to an oxime polymer, which is a polymer matrix modified with levulinic acid oxime of the general formula: 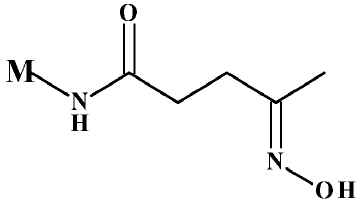 where M - polymer matrix.

## Description

### Field of the Invention

The invention relates to organic chemistry, in particular, to a novel oxime-containing polymer that can be used for the synthesis of polypeptides containing various functional groups on the C-terminus.

Synthetic polypeptides are widely used as bases in the development of various types of medications, vaccines, and materials for medical use.

### Prior Art

One of the most effective industrial methods for the preparation of polypeptides is solid-phase synthesis [see, for example, B.W. Erikson and R.B. Merrifield, Proteins, 3 255-527 (1976)]. In this case, the first amino acid residue is attached to an insoluble polymer carrier after which acetylation and unblocking stages are repeated until a desired sequence length is obtained. The peptide is then cleaved from the polymer carrier while simultaneously removing protective groups with strong acids, purified in a chromatograph, and freeze dried.

A large number of polymer carriers used in solid-phase synthesis has been described in the literature. In addition to the type of the polymer matrix and the degree of its cross-linking, the structure of the used linker group is of utmost importance. A linker group, i.e. the functional group that links an amino acid residue to a polymer, defines the way a peptide connects to a polymer carrier, conditions under which it is cleaved, and whether the finished product is obtained as a free acid, amide, substituted amide, hydrazide, ester, etc. Considerable attention is also paid to the development of effective peptide cyclization methods on a polymer because cyclopeptides are largely resistant to enzymatic cleavage and also, in some cases, are more biologically active than linear peptides.

Thus, selection of a polymer carrier and, in particular, the structure of the linker group determines the efficacy of using said carrier for the synthesis of biologically active peptides or reactive blocks in the subsequent peptide elongation.

Especially noteworthy is Kaiser's polymer, which contains p-nitrobenzophenone oxime as the linker group [W.F. DeGrado and E.T. Kaiser, J. Org. Chem. 1980, 45, 1295 - 1300]: 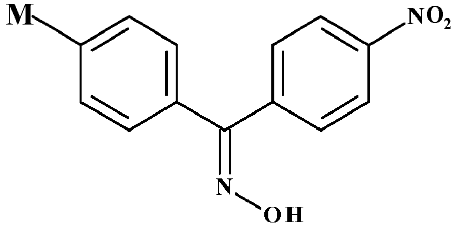 where M - polymer matrix; in this case, the cross-linked styrene-divinylbenzene copolymer.

Kaiser's polymer was successfully used for synthesizing cyclic peptides and preparing linear polypeptides as amides, esters, and hydrazides. Kaiser's polymer, however, has a number of disadvantages, such as:
- The use of toxic reagents, such as nitrobenzene and ethylene dichloride in the synthesis of the starting polymer carrier;
- During peptide synthesis, Kaiser's polymer is insufficiently resistant to trifluoracetic acid used to remove the temporary protective tert-butyloxycarbonyl group; partial stability of the peptide-polymer bond during deblocking of the amino group results in limiting the length of synthesized peptides due to their gradual cleavage from the polymer carrier;
- During the synthesis of polypeptides using Kaiser's polymer, peptides can be cleaved from the polymer carrier by alkali, which eliminates the option of preliminary neutralization, and the alkali has to be added at the stage of the peptide bond formation, which limits the choice of the applicable synthetic techniques;
- Resistance of the peptide-polymer bond to alkalis prevents its use in the synthesis where such alkali-liable protective groups as 9-fluorenylmethoxycarbonyl are used.

### Summary of the Invention

The object of this invention is a novel oxime-containing (oxime) polymer, a polymer carrier for the synthesis of linear and cyclical polypeptides containing various functional groups at the C-terminus of the peptide chain.

An additional objective is to simplify the oxime polymer preparation while using readily available starting compounds and reagents and eliminating the need of using toxic solvents, such as nitrobenzene and ethylene dichloride.

The stated objectives are achieved with the proposed oxime polymer, which is a polymer matrix modified with levulinic acid oxime of the general formula: 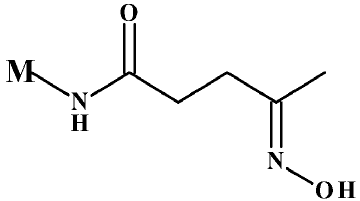 wherein M - polymer matrix.

To prepare the oxime polymer, we used levulinic (4-oxopentanoic) acid.

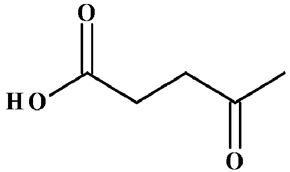

The polymer matrix can be styrene-divinylbenzene copolymer; a similar polymer with grafted polyethylene glycol; polyethylene, cross-linked polyethylene glycol; acrylamide-based polymers, and many other copolymers. Additionally, in order to be used in the synthesis, the polymer matrix must contain an amino group.

The claimed oxime polymer can be prepared by acylating the amino group in the aminomethylated styrene-divinylbenzene copolymer or other amino-group-containing copolymers with levulinic acid in the presence of condensing agents, such as diisopropylcarbodiimide, dicyclohexylcarbodiimide, 2-(6-chloro-1-H-benzotriazole-1-yl)-1,1,3,3-tetramethyluronium hexafluorophosphate (HCTU), N,N,N',N'-tetramethyl-O-(1H-benzotriazole-1-yl)uronium tetrafluoroborate (TBTU), N,N,N',N'-tetramethyl-O-(1H-benzotriazole-1-yl)uronium hexafluoroborate (HBTU), and others used in solid-phase peptide synthesis, followed by treatment with hydroxylamine hydrochloride in the presence of pyridine.

Example 1 demonstrates the synthesis of the oxime polymer by modifying a styrene-divinylbenzene-copolymer-based matrix. Examples 2-4 show resistance of the claimed oxime polymer to trifluoroacetic acid and alkalis as well as examine the possibility of racemization during the cleavage of the peptide from the polymer carrier.

### Example 1. Synthesis of N-levulinoyl oxime aminomethyl polystyrene polymer

7 g of the aminomethyl polystyrene polymer (IRIS Biotech GmbH; capacity 0.56 mmol/g) were kept in 50 ml of dichloromethane for one hour. The solvent was filtered off, 1.34 g (11.76 mmol, 3 equiv.) of levulinic acid dissolved in 35 ml of dichloromethane were added to the polymer; the mixture was stirred for 10 min., and 1.82 ml (11.76 mmol, 3 equiv.) of N,N'- diisopropylcarbodiimide were added to the mixture. The polymer was stirred for one hour and left overnight at room temperature. The reaction mixture was filtered and the polymer was successively washed (3 times) with dichloromethane, dimethylformamide, and ethanol. Completion of the reaction was monitored by the Kaiser Test (Ninhydrin test) [Kaiser E., Colescott R.L., Bossinger C.D., Cook P.I., Anal. Biochem. 34: 595, 1970]).

70 ml of ethanol, 7 g of hydroxylamine hydrochloride, and 7 ml of pyridine were added to the levunoyl aminomethyl polystyrene polymer obtained in the previous stage. The polymer suspension was boiled under reflux for 6 hrs. and left overnight at room temperature. The polymer was filtered and successively washed twice with ethanol, twice with 50% aqueous ethanol, 3 times with dimethylformamide, twice with ethanol, and twice with ether. Drying in vacuo yielded 7.5 g of the polymer.

IR data obtained on an IR Fourier spectrometer (Vertex 70; Bruker) show an absorption band at 1716 cm⁻¹ (keto-group C=O band) after levulinic acid has been attached to the initial polymer. This band disappears with the addition of hydroxylamine producing an oxime.

Determination of the polymer's capacity (the number of functional groups per gram of the polymer) by loading 9-fluorenylmethoxycarbonyl alanine, deblocking, and spectrophotometrically determining the amount of the formed dibenzofulvene [Gude M., Ryf J., White P.D., Letters in Peptide Science, 9: 203, 2002] show that the modification is practically 100% complete.

### Example 2. Determining resistance of the oxime polymer to trifluoroacetic acid

A solution of 9- fluorenylmethoxycarbonyl alanine (0.174 g; 0.56 mmol) in 2 ml of dichloromethane was added to 200 g of the oxime polymer, which had been previously washed with dichloromethane, and stirred for 10 min. N,N'-diisopropylcarbodiimide (0.87 mcl; 0.56 mmol) was added to the reaction mixture and the solution was left for 8 hrs. The solvent was filtered off, and the polymer was washed twice with dimethylformamide, twice with methylene chloride, and 3 times with ether, then dried in a vacuum desiccator. Capacity of the polymer was determined according to the procedure described in Example 1.

The loaded polymer was held in trifluoroacetic acid for 3 hrs., washed with methylene chloride 3 times, and 3 times with ether, then dried in a vacuum desiccator. The polymer capacity determined according to the procedure described in Example 1 showed no changes within the experimental error limits.

### Example 3. Determining resistance of the oxime polymer to 1,8-diazabicyclo(5.4.0)undec-3-ene

The polymer obtained according to the procedure described in Example 2 was treated with 2% solution of 1,8-diazabicyclo(5.4.0)undec-3-ene in dimethylformamide (1x2 min and 1x8 min) and washed twice with dimethylformamide, twice with isopropyl alcohol, and 3 times with dimethylformamide. A solution of 9-fluorenylmethoxycarbonyl phenylalanine (194 mg; 0.5mmol) and ethyl cyanohydroxy-iminoacetate (71 mg; 0.5 mmol) in dimethylformamide was added to the deblocked polymer. N,N'-diisopropylcarbodiimide (0.77 mcl; 0.5mmol) was added to the reaction mixture with stirring and the resulting mixture was left for 8 hrs. The solvent was filtered off and the polymer was washed 3 times with dimethylformamide and 3 times with ether, after which it was dried in a vacuum desiccator.

The polymer capacity determined according to the procedure described in Example 1 showed no changes within the experimental error limits.

### Example 4. Analyzing the possibility of racemization under conditions of peptide's cleavage from a polymer carrier

An important condition for the synthesis of biologically active polypeptides is preservation of optical purity of amino acid residues (absence of racemization). To confirm the absence of racemization during the peptide's cleavage from the oxime polymer, a highly sensitive test proposed by F. Albericio [Subirós-Funosas R., Prohens R., Bar-bas R., El-Faham A., Albericio F., Chem. Eur. J., 15: 9394, 2009] was applied. The test is based on the condensation reaction between benzyloxycarbonyl-phenylalanyl-valine and proline amide followed by separation of the formed diastereoisomers by reversed-phase high-performance liquid chromatography (RP-HPLC) Z-Phe-Val-OH + H-Pro-NH₂ Z-Phe-Val-Pro-NH₂. 250 mg of the oxime polymer containing benzyloxycarbonyl-phenylalanyl-valine attached thereto and suspended in 2 ml of tetrahydrofuran was combined with 5 eqv. of proline amide and kept for 8 hrs. The solvent was evaporated and the product was analyzed by RP-HPLC using benzyloxycarbonyl-L-phenylalanyl-L-valyl-L-prolineamide and benzyloxycarbonyl-L-phenylalanyl-D-valyl-L-prolineamide as fiducial markers.

Results of the analysis confirm the absence of any significant impurities of benzyloxycarbonyl-L-phenylalanyl-D-valyl-L-prolineamide, which leads to the conclusion that no racemization takes place when the peptide cleaves from the polymer carrier during aminolytic splitting.

### Example 5. Synthesis of model tripeptides BOC-Gly-Phe-Ala-OH and BOC-Gly-Phe-Ala-OMe

A possible application of the claimed polymer in the synthesis of peptides and their derivatives was evaluated in the example of preparation of tert-benzyloxycarbonyl-glycyl-phenylalanyl-alanine (BOC-Gly-Phe-Ala-OH) as a compound with a free carboxy- group and as the corresponding methyl ether (BOC-Gly-Phe-Ala-OMe).

The initial peptidyl polymer was prepared by standard solid-phase methods of peptide synthesis. Tert-benzyloxycarbonyl protection was removed with 25% trifluoroacetic acid solution in methylene chloride; the second and third amino acid residues were added using 2-(6-chloro-1-H-benzotriazol-1-il)-1,1,3,3-tetramethyluronium hexafluorophosphate (HCTU) in dimethylformamide in the presence of diisopropylethylamine.

The peptide was cleaved from the polymer carrier with 140 mg of the peptidyl polymer and 42 mcl 1,8-diazabicyclo(5.4.0)undec-3-ene in 2 ml of tetrahydrofuran-water mixture (95:5 when preparing a peptide with a free carboxy-group) and in 2 ml of the tetrahydrofuran-methanol mixture (95:5 when preparing the peptide methyl ether).

An analogous (control) synthesis was conducted on Kaiser's polymer.

Purity of the prepared raw product (unpurified peptide) was evaluated by reversed-phase high-performance liquid chromatography. The obtained results are shown in the table below:

**Content of the end product %**

| Amino acid sequence | Kaiser's Polymer | Claimed Polymer |
|---|---|---|
| BOC-Gly-Phe-Ala-OH | 72 | 81 |
| BOC-Gly-Phe-Ala-OMe | 82 | 88 |

As the above data demonstrate, the claimed oxime-containing copolymer can be synthesized from readily available initial compounds and reagents, and moreover, its synthesis does not require the use of such toxic solvents as nitrobenzene and dichloroethane.

Strong resistance of the claimed oxime-containing polymer to trifluoroacetic acid eliminates polypeptide synthesis limitations when using the acid-liable tert- benzyloxycarbonyl (BOC) protection.

Because it is resistant to such deblocking agents as 1,8-diazabicyclo(5.4.0)undec-3-ene (DBU), the alkali-liable 9-fluorenylmethoxycarbonyl (Fmoc) protection can be used, at least at some stages of the synthesis.

Moreover, no significant racemization was observed at the cleavage stage of the final product from the polymer carrier.

Because of all that, the claimed oxime polymer can be successfully used for the preparation of polypeptides and their derivatives containing various C-terminus functional groups.

### Industrial applicability

The claimed oxime-containing polymer can be used in the industrial synthesis of polypeptides containing various C-terminus functional groups, which are widely used as bases in the development of various types of medications, vaccines, and materials for medical use.

## Claims

1. n oxime polymer, which is a polymer matrix modified with levulinic acid oxime of the general formula: 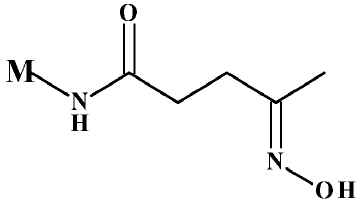 where M - polymer matrix.
